# EUROPEAN PATENT APPLICATION

(11) **EP 4 847 033 A1**
(43) Date of publication of application: **02.12.2026**
(21) Application number: 26180708.5
(22) Date of filing: 22.05.2026
(51) Int. Cl.: A61K 38/08, A61K 38/26, A61P 3/04

(54) **MEDICATION FOR USE IN TREATING OBESITY COMPRISING PEPTIDE**

(30) Priority: 27.05.2025 US 202563812216 P; 04.09.2025 US 202563875351 P
(71) Applicant: Pell Bio-Med Technology Co., Ltd., Taipei City (TW)
(72) Inventor: LIN, Chen-Lung, Taipei City (TW)
(74) Representative: Tiburzi, Andrea

(57) **Abstract**

Provided is a medication for use in treating obesity and comprises a peptide; wherein the peptide comprises an amino acid sequence set forth in SEQ ID NO: 1, and the effective amount of the peptide is more than 0.5 mg/kg/day. The present disclosure verifies that administering the peptide of SEQ ID NO: 1 within the specific dose range can inhibit the increase of body weight caused by high-fat diet, thereby providing a therapeutic effect for obesity.

## Description

The instant disclosure relates to a medication for use in treating obesity comprising a peptide, particularly to a medication for use in treating obesity comprising a peptide set forth in SEQ ID NO: 1.

Social development and technological progress, though elevating the convenience and quality of life, lead to many chronic diseases. Obesity is a common but easily ignored dangerous sign, and World Health Organization (WHO) and U.S. Food and Drug Administration (FDA) have recognized obesity as one of the chronic diseases since 1996.

Obesity indicates a disease that causes excessive accumulation of body fat and increment of weight due to changes in physiological or biochemical functions, and the main causes of obesity are generally considered as unhealthy diet and insufficient exercise. In general, obesity is classified to simple obesity (or primary obesity) and secondary obesity, and most cases of obesity belong to simple obesity.

Simple obesity can be further classified to constitutional obesity and acquired obesity, wherein constitutional obesity indicates a condition caused by a large number of fat cells, which is common in childhood; and acquired obesity indicates a condition caused by increased volume of fat cells, which is common in adulthood. On the other hand, secondary obesity is caused by endocrine or metabolic diseases, and is also called symptomatic obesity.

As obesity further causes other diseases such as cardiovascular disease, diabetes, hypertension or even cancer, etc., the treatment of obesity for lowering the risk of subsequent diseases has become one of the research focuses in the relevant technical fields. Common research directions include: promoting degradation of triglyceride (TG) in fat tissue for reducing body fat; inhibiting digest and absorption functions for reducing absorbing substances required for body fat formation; or lowering appetite and/or delaying gastric emptying for increasing satiety and reducing calorie intake.

Various drugs for treating obesity are available on the market, such as liraglutide. Liraglutide is an agonist targeting the receptor of glucagon-like peptide-1 (GLP-1), which inhibits human appetite center to reduce appetite and delays gastric emptying to increase satiety, thereby achieving the effect of treating obesity. However, as the current proportion of obesity population remains high, it is necessary to further research and develop more drugs that can provide therapeutic effects on obesity.

In light of the needs in the prior art, an objective of the present disclosure is to provide a medication for use in treating obesity comprising a peptide, and the peptide demonstrates an effect of inhibiting weight increment caused by high-fat diet, thereby providing more therapeutic options for obesity patients.

To achieve the foresaid objective, the present disclosure provides a medication for use in treating obesity, which comprises a peptide; wherein the peptide comprises an amino acid sequence set forth in SEQ ID NO: 1, and the effective amount of the peptide is more than 0.5 mg/kg/day (*i.e.*, 0.5 milligrams per kilogram per day).

By adopting the peptide comprising the amino acid sequence set forth in SEQ ID NO: 1 and controlling the amount of the peptide within the specific range of the effective amount of the peptide at the same time, the medication exerts an effect of inhibiting weight increment caused by high-fat diet, thereby providing therapeutic effect for obesity.

In some embodiments, the foresaid peptide is the amino acid sequence set forth in SEQ ID NO: 1.

In some embodiments, the medication comprising the peptide is administered to a subject in need with the effective amount of the peptide.

In some embodiments, the effective amount of the peptide may be more than or equal to 0.5 mg/kg/day and less than or equal to 8 mg/kg/day. In some embodiments, the effective amount of the peptide may be more than or equal to 1 mg/kg/day and less than or equal to 5 mg/kg/day. In some embodiments, the effective amount of the peptide may be more than or equal to 1 mg/kg/day and less than or equal to 10 mg/kg/day. In some embodiments, the effective amount of the peptide may be more than or equal to 2 mg/kg/day and less than or equal to 9 mg/kg/day. It is understood that the foresaid effective amount is for humans, and is obtained by a method called "Estimating the maximum safe starting dose in initial clinical trials for therapeutics in adult healthy volunteers" published by FDA in 2005, which describes that based on an adult having a body weight (b.w.) of 60 kilograms, 12.3-fold of a suggested intake amount for humans per kilogram per day (/kg b.w./day) equals to 1-fold for mice. For example, when an effective amount for mice is 15 mg/kg/day, the effective amount for humans will be about 1.2195 mg/kg/day (or ranging from about 1.2 mg/kg/day to 1.3 mg/kg/day) based on the foresaid conversion.

In some embodiments, an administration frequency of the peptide may be from once per day to once per month. For example, the administration frequency of the peptide may be, but is not limited to, once per day, twice per day, once per week, twice per week or three times per week.

In some embodiments, the medication is administered in combination with a GLP-1 receptor agonist. By adopting the medication comprising the peptide in combination with the GLP-1 receptor agonist, and controlling the amount of the peptide within the specific range of the effective amount of the peptide at the same time, the combination of the medication comprising the peptide and the GLP-1 receptor agonist exerts an effect of inhibiting weight increment caused by high-fat diet, thereby providing therapeutic effect for obesity.

In some embodiments, when the medication is administered in combination with the GLP-1 receptor agonist, the GLP-1 receptor agonist may be included in the medication together with the peptide; or the GLP-1 receptor agonist may be included in another independent medication that does not comprise the peptide.

In some embodiments, the medication may be a pharmaceutical combination, which comprises the peptide and a GLP-1 receptor agonist.

In some embodiments, the peptide and the GLP-1 receptor agonist may be separately administered to a subject in need by the same or different administration routes.

In some embodiments, the peptide and the GLP-1 receptor agonist may be simultaneously or sequentially administered to a subject in need by the same or different administration routes.

In some embodiments, the peptide and the GLP-1 receptor agonist may be administered at the same or different administration frequencies.

In some embodiments, an administration frequency of GLP-1 receptor agonist may be from once per day to once per month. For example, the administration frequency of the GLP-1 receptor agonist may be, but is not limited to, once per day, twice per day, once per week, twice per week or three times per week.

In some embodiments, an effective amount of the GLP-1 receptor agonist may be more than 1 µg/kg/day. In some embodiments, an effective amount of the GLP-1 receptor agonist may be more than or equal to 2 µg/kg/day and less than or equal to 20 µg/kg/day. In some embodiments, an effective amount of the GLP-1 receptor agonist may be more than or equal to 2 µg/kg/day and less than or equal to 10 µg/kg/day. In some embodiments, an effective amount of the GLP-1 receptor agonist may be more than or equal to 2 µg/kg/day and less than or equal to 5 µg/kg/day.

In some embodiments, the GLP-1 receptor agonist may be dulaglutide, liraglutide, lixisenatide, semaglutide, tirzepatide, danuglipron, orforglipron, or any combination thereof. In some embodiments, the GLP-1 receptor agonist may be liraglutide.

In some embodiments, the foresaid obesity may be simple obesity.

In some embodiments, the foresaid obesity may be acquired obesity.

In some embodiments, the medication comprises the peptide and a pharmaceutically acceptable carrier. In some embodiments, the medication comprises the peptide, the GLP-1 receptor agonist and a pharmaceutically acceptable carrier. The pharmaceutically acceptable carrier may comprise a solvent, an emulsifier, a suspending agent, a decomposer, a binding agent, an excipient, a stabilizing agent, a diluent, a gelling agent, a lubricant, a surfactant, or other carriers similar or suitable to the carrier of the present disclosure, or any combination thereof, but it is not limited thereto.

In some embodiments, the peptide is accompanied with a first pharmaceutically acceptable carrier; and the GLP-1 receptor agonist is accompanied with a second pharmaceutically acceptable carrier. The first pharmaceutically acceptable carrier and the second pharmaceutically acceptable carrier may independently comprise a solvent, an emulsifier, a suspending agent, a decomposer, a binding agent, an excipient, a stabilizing agent, a diluent, a gelling agent, a lubricant, a surfactant, or other carriers similar or suitable to the carrier of the present disclosure, or any combination thereof, but it is not limited thereto.

In some embodiments, the medication or the pharmaceutical combination may be in an enteral dosage form or a parenteral dosage form. The enteral dosage form may comprise, but is not limited to, an oral dosage form comprising an enteric coated tablet, a multilayer tablet, a sugar-coated tablet, a sublingual tablet, a chewable tablet, a troche, a capsule, a powder, a syrup, a solution, an emulsion, a suspension, a mucilage, a magma, a fluid extract, an extract, a spirit, an elixir, and a tincture, etc.; and enema. The parenteral dosage form may comprise, but is not limited to, a dosage form for injection, an ointment, a lotion, a liniment, and an aerosol.

Preferably, the enteral dosage form may be an oral dosage form.

Preferably, the parenteral dosage form may be a dosage form for injection.

In some embodiments, the peptide may be administered by enteral administration or parenteral administration. In some embodiments, the peptide may be administered by enteral administration. In some embodiments, the peptide may be administered by oral administration.

In some embodiments, the GLP-1 receptor agonist may be administered by enteral administration or parenteral administration. In some embodiments, the GLP-1 receptor agonist may be administered by parenteral administration. In some embodiments, the GLP-1 receptor agonist may be administered by subcutaneous administration.

According to the present disclosure, the terms "treating" and "treatment" refer to administering the effective amount of the peptide to a patient in order to achieve the effects of curing, mitigating, ameliorating or relieving a disease or related symptoms. Specifically, the terms "treating obesity" and "treatment of obesity" of the present disclosure may comprise, but are not limited to, inhibiting weight increment caused by high-fat diet.

According to the present disclosure, the term "effective amount" refers to a dosage for the desired biological reaction, namely, a dosage for the desired treatment effect.

Other objectives, advantages and novel features of the instant disclosure will become more apparent from the following detailed description when taken in conjunction with the accompanying drawings.
In the drawings:
FIG. 1 shows the results of average body weight measured at different timepoints of mice fed with normal diet or mice fed with high-fat diet in Test Example 1.
FIG. 2 shows the results of measured contents of serum TC, serum TG, serum LDL-C and serum HDL-C on day 91 of mice fed with normal diet or mice fed with high-fat diet in Test Example 1.
FIG. 3 shows the results of measured fat mass and lean mass on day 91 of mice fed with normal diet or mice fed with high-fat diet in Test Example 1.
FIG. 4 shows the results of body weight change at different timepoints of mice treated with different conditions in Test Example 1.
FIG. 5 shows the results of body weight change of mice treated with different conditions on day 28 of each group in Test Example 1.
FIG. 6 shows the results of body weight change at different timepoints of mice treated with different conditions in Test Example 2.

The following Examples and Comparative Examples are provided to illustrate the implementation of the present disclosure. A person skilled in the art will readily recognize the advantages and effects of the present disclosure based on the contents of this specification.

### Preparation Example 1: Preparation of peptide set forth in SEQ ID NO: 1

The peptide used in the present disclosure is the same as that described in Taiwan Patent Publication No. I705818. Specifically, the standard Fmoc strategy and microwave peptide synthesizer were used to synthesize the peptide of the present disclosure, which had an amino acid sequence of: Glp Glu Thr Ala Val Ser Ser His Glu Gln Asp, wherein the Glp was pyroglutamic acid (Cas Number: 98-79-3). In general, Wang resin (0.6 mmol/g of load) preloaded with D residues was loaded into a reaction vessel, and 10 mL to 15 mL of fresh dimethylformamide (DMF) was added ready to be swollen before the synthesis. The swelling time was set as 3 minutes (min) on the microwave peptide synthesizer.

The first and subsequent Fmoc groups were removed by 5 mL of 20% Piperidine in DMF by a standard de-protection process (as the "DEP" step) (in the first stage, the temperature was 75°C, the power was 155 Watts (W), and the temperature-holding time was 15 seconds; in the second stage, the temperature was 90°C, the power was 30 W, and the temperature-holding time was 50 seconds), in order to free the N-terminal amine group.

Then, 0.5 M N,N'-diisopropylcarbodiimide (DIC) in DMF was added to activate C terminus (as the "ACT" step), and the desired amino acid (as the Fmoc-AA (protected side chain)-OH) in a five-fold excess amount (with a concentration of 0.2 M in DMF) and 1.0 M Oxyma in DMF were added for coupling reaction (as the "AA" step). The standard coupling reaction was processed (in the first stage: the temperature was 75°C, the power was 170 W, and the temperature-holding time was 15 seconds; in the second stage: the temperature was 90°C, the power was 30 W, and the temperature-holding time was 230 seconds). After conducting the "AA" step for about 4 minutes, the Wang resin was washed with DMF solution. The steps of DEP-ACT-AA were repeated to construct a peptide from C-terminus to N-terminus.

Finally, the peptide was removed from the solid support by treating with 95% TFA/2.5% H2O/2.5% TIPS in an ice bath and allowing the reaction to come to room temperature for 2 hours. The filtrate was collected after filtration, and an iced ether was added for precipitation and then centrifuged to remove supernatant. After that, ether was added again to wash the precipitate. The aforementioned steps were repeated for 3 to 6 times. The final precipitate was taken out and freeze-dried to obtain a white floccus-like product, which is the final product. The purity of the final product was determined by high performance liquid chromatography. The molecular weight and the sequence of the synthesized peptide were analyzed by Nano LC-MS/MS, thereby obtaining the peptide of SEQ ID NO: 1.

### Test Example 1: Evaluation of the effect of the peptide of the present disclosure for treating obesity with animal experiment

### (1) Building mice model with obesity induced by high-fat diet

Male mice with strain of C57BL/6 (purchased from Lingchang Biological Technology Co., Ltd., Shanghai) were adopted, and when the mice were at 6 to 7 weeks age, high-fat diet (purchased from Research Diets, Inc; product number: D12492) or normal diet (purchased from Research Diets, Inc; product number: D12450J) were respectively used to feed different groups of mice for 14 weeks, so the mice fed with high-fat diet were induced to have obesity, and the mice with high-fat diet-induced obesity (DIO) were obtained. During the 14-week induction period, mice body weights were measured on day 0, day 7, day 14, day 21, day 28, day 35, day 42, day 49, day 56, day 63, day 70, day 77, day 84, day 91 and day 98, respectively. Besides, on day 91, 30 microliters of mice serum were collected and the total cholesterol (TC), triglyceride (TG), low-density lipoprotein cholesterol (LDL-C) and high-density lipoprotein cholesterol (HDL-C) in the serum were measured; meanwhile, the mice were scanned by a non-invasive body composition analyzer EchoMRI to obtain the results of whole-body composition, which comprised fat mass and lean mass (without fat weight). Then, based on the mice body weight on day 98, the component concentration in the serum and whole-body composition on day 91, 6 of mice fed with normal diet were grouped as normal diet group and 38 of mice fed with high-fat diet were grouped as high-fat diet group for the usage of subsequent experiments, wherein the mice in each group had no obvious differences on the mice body weight, component concentration in the serum and whole-body composition.

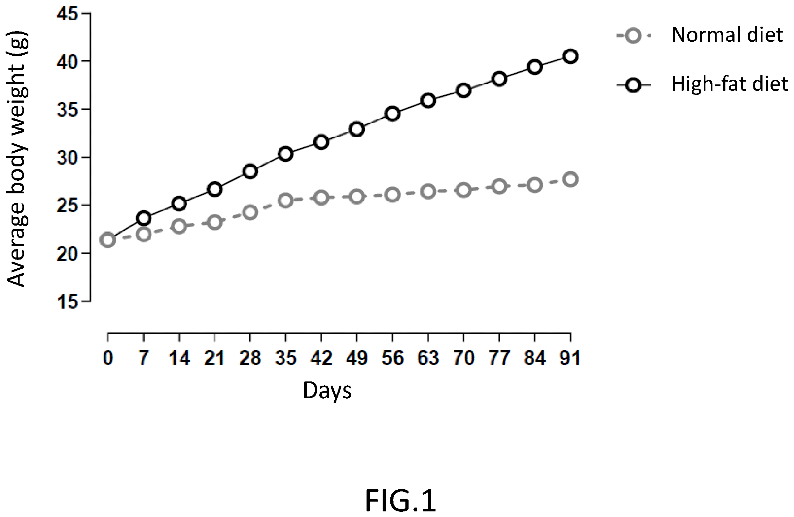

The results of body weight measurements at different timepoints of mice fed with normal diet or high-fat diet during the foresaid 14-week induction period were as shown in FIG. 1 (the result on day 98 was not shown). In FIG. 1, the body weights of mice fed with high-fat diet were obviously increased over time, generally higher than the body weights of mice fed with normal diet. Besides, on day 91, the average body weight of mice fed with high-fat diet was about 40.5 grams, and the average body weight of mice fed with normal diet was about 27.7 grams only.

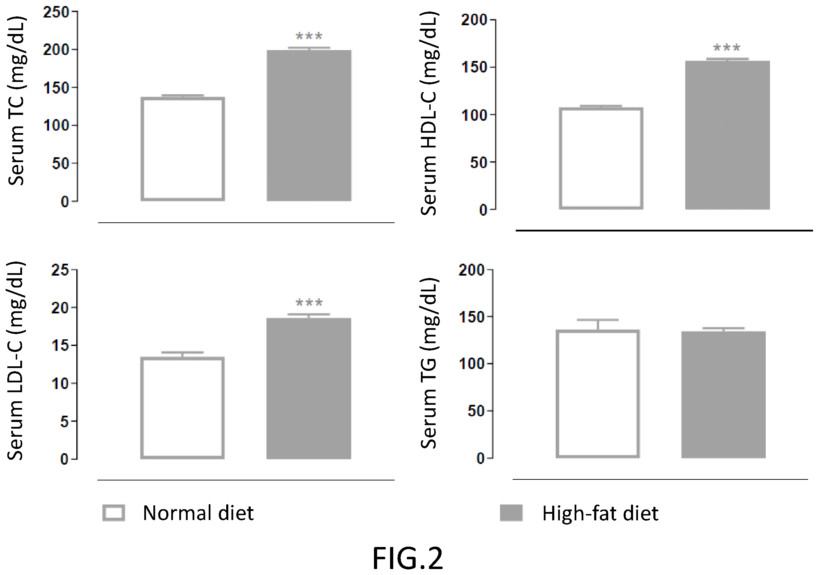

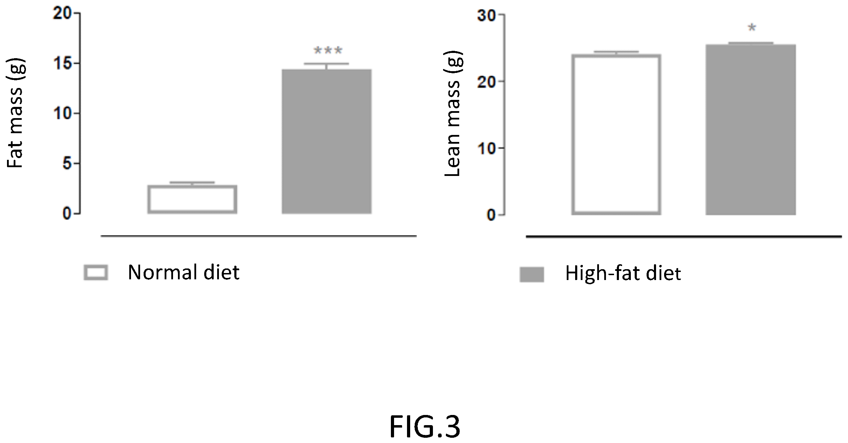

In addition, the results of serum TC, serum TG, serum LDL-C and serum HDL-C measurements of mice fed with normal diet or high-fat diet on day 91 were shown in FIG. 2; and the results of the foresaid fat mass and lean mass measurements were shown in FIG. 3. In FIG. 2, the contents of serum TC, serum LDL-C and serum HDL-C of mice fed with high-fat diet were higher than those of mice fed with normal diet, which showed statistically significant difference with *p*-value less than 0.001. In FIG. 3, the fat mass of mice fed with high-fat diet was obviously much higher than that of mice fed with normal diet, which showed statistically significant difference with *p*-value less than 0.001; and the lean mass of mice fed with high-fat diet was obviously higher than that of mice fed with normal diet, which showed statistically significant difference with *p*-value less than 0.05. The foresaid statistic results were analyzed and obtained by two-tailed Student's t-test.

Accordingly, based on the results in FIG. 1, FIG. 2 and FIG. 3, mice having obesity were successfully obtained by induction with the high-fat diet for 14 weeks.

### (2) Animal experiment

### (I) Treating conditions

After mice of the normal diet group and mice of the high-fat diet group (having obesity) were obtained according to the foresaid processes, the peptide of Preparation Example 1 was dissolved in deionized water (as a solvent) and then 1.5 mg/mL of sodium bicarbonate solution was slowly added until the solution was clear, followed by adding deionized water to predetermined concentration to obtain a sample of the peptide of Preparation Example 1 (pH=5 to 6). Then, different amounts of the samples were administered to the mice of the high-fat diet group for evaluating the effect of obesity treatment of the peptide. The treating conditions such as sample administration dosages, administration routes, administration conditions and administration time, etc. for different groups were listed in Table 1, including Example 1 (high-fat diet), Example 2 (high-fat diet) and Comparative Example 1 (high-fat diet). Besides, Table 1 also listed the treating conditions of the following groups: Solvent group 2 (high-fat diet) replaced the sample with deionized water, Control group 1 (high-fat diet) replaced the sample with liraglutide (purchased from MedChemExpress LLC; product number: HY-P0014) and Solvent group 1 (normal diet) fed mice of the normal diet group with deionized water. In Table 1, "DIW" meant "deionized water", "P" meant "the sample of peptide of Preparation Example 1 dissolved in deionized water", "L" meant "liraglutide dissolved in 0.9% saline", "N" meant "mouse number" and "SC" meant "subcutaneous injection".

**Table 1: treating conditions for mice of Solvent group 1 (normal diet), Solvent group 2 (high-fat diet), Example 1 (high-fat diet), Example 2 (high-fat diet), Comparative Example 1 (high-fat diet) and Control group 1 (high-fat diet)**

| | | | | | | |
|---|---|---|---|---|---|---|
| Group | Sample | Dosage | N | Administration | | |
| | | | | Route | Frequency | Time |
| Solvent group 1 (normal diet) | DIW | 5 mL/kg | 6 | Oral | Twice per day at 8-hour intervals | 28 days |
| Solvent group 2 (normal diet) | | 5 mL/kg | 6 | Oral | | |
| Example 1 (high-fat diet) | P | 15 mg/kg | 8 | Oral | | |
| Example 2(high-fat diet) | | 50 mg/kg | 8 | Oral | | |
| Comparative Example 1 (high-fat diet) | | 5 mg/kg | 8 | Oral | | |
| Control group 1 (high-fat diet) | L | 200 µg/kg | 8 | SC | | |

### (II) Therapeutic effect evaluation

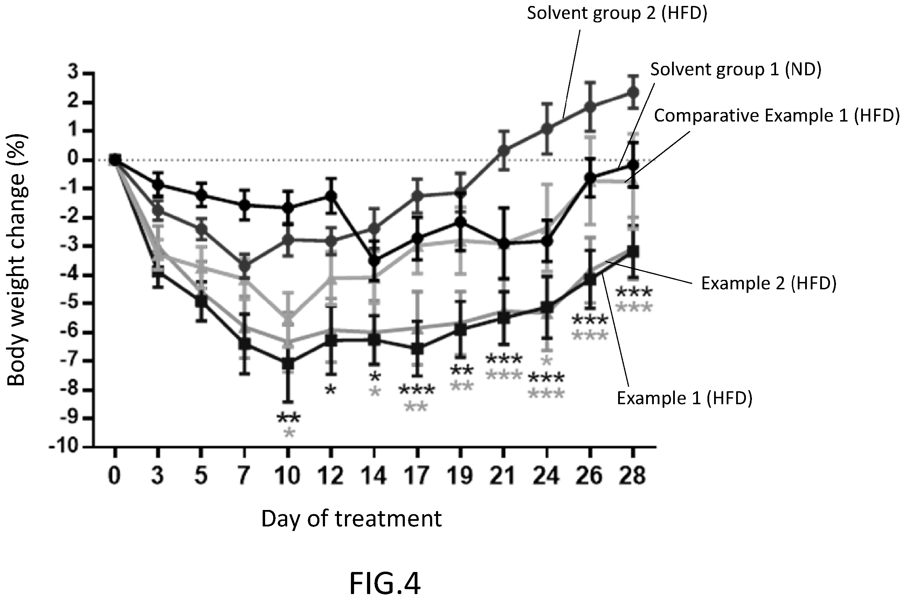

During the process of treating mice of each group according to the treating conditions in Table 1, mice body weights were measured on day 0, day 3, day 5, day 7, day 10, day 12, day 14, day 17, day 19, day 21, day 24, day 26 and day 28, respectively. Then, average body weight on the foresaid each timepoint was calculated, and the body weight change (%) at each timepoint was calculated (the average body weight at each timepoint subtracted the average body weight on day 0, followed by a percentage conversion) for evaluating the effect of obesity treatment, and the results were shown in FIG. 4, wherein HFD represented high-fat diet and ND represented normal diet. The results of each group in FIG. 4 were statistically analyzed by two-way repeated measures ANOVA accompanied with Bonferroni's post hoc test, and "*" meant p-value less than 0.05, "**" meant *p*-value less than 0.01 and "***" meant *p*-value less than 0.001.

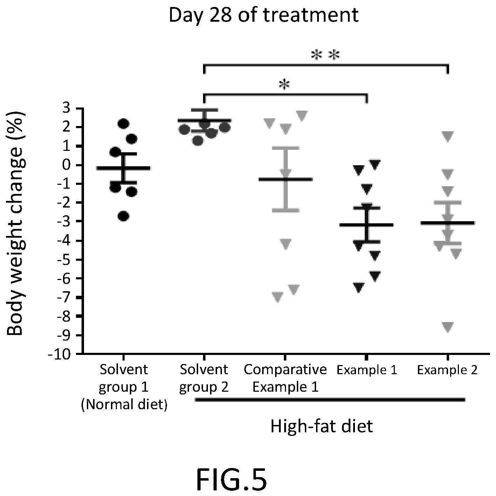

Besides, body weight change on day 28 of treatment of each mouse in groups of Solvent group 1 (normal diet), Solvent group 2 (high-fat diet), Comparative Example 1 (high-fat diet), Example 1 (high-fat diet) and Example 2 (high-fat diet) were further analyzed in FIG. 5, and the results of each group were statistically analyzed by two-tailed Student's t-test, and "*" meant p-value less than 0.05 and "**" meant *p*-value less than 0.01.

In addition, the difference of the average body weights of mice in Control group 1 (high-fat diet) between day 28 and day 0 was about 26% (not shown in FIG. 4 and FIG. 5), which meant that the body weight change of Control group 1 (high-fat diet) on day 28 of treatment was about -26%.

### (III) Discussion for experimental results

Referring to FIG. 4, the body weight change of Solvent group 1 (ND) slightly decreases at early stage (day 14), and then gradually rises. On day 28, the body weights of mice are almost back to the body weight on day 0. It indicates that administering mice fed with normal diet with deionized water does not significantly affect the body weight, and the foresaid change of body weight should result from normal weight fluctuation (such as, gavage feeding of mice usually causes a slight decrease in weight in the initial stage) or unavoidable measurement errors, etc.

For the mice of Solvent group 2 (HFD), the body weight also slightly decreases at early stage (day 7), and the degree of decrease is similar to Solvent group 1 (ND). Notably, the body weight begins to rise gradually from day 10, the body weight on day 21 is almost back to the body weight on day 0, and the body weight change on day 28 is about 2.4%. It indicates that administering mice fed with high-fat diet with deionized water does not obviously inhibit body weight increment, and the body weight of mice fed with high-fat diet gradually rises indeed.

For the mice of Comparative Example 1 (HFD), the body weight shows obvious decrease (about -5.5% of body weight change) at early stage (day 10), while the body weight gradually rises afterward and shows a trend similar to Solvent group 2 (HFD). Although the body weight change of Comparative Example 1 (HFD) on day 28 is about -0.8%, it shows no statistically significant difference compared to the result of Solvent group 2 (HFD). In contrast, for the mice of Example 1 (HFD) and Example 2 (HFD), the body weights of these groups show obvious decrease (about -6% to -7% of body weight change) at early stage (day 10), and the body weight changes are maintained at about -6% to -7% from day 12 to day 19, and the body weights begin to rise gradually after day 21. On day 28, the body weight change of Example 1 (HFD) is still about -3.2% and the body weight change of Example 2 (HFD) is still about -3.1%, and both groups show statistically significant difference compared to the result of Solvent group 2 (HFD) with *p*-value less than 0.001. Besides, in comparison with the results of Solvent group 2 (HFD), the results of Example 1 (HFD) on day 12 and day 14 show statistically significant difference with *p*-value less than 0.05, on day 10 and day 19 show statistically significant difference with *p*-value less than 0.01 and on day 17, day 21, day 24, day 26 and day 28 show statistically significant difference with *p*-value less than 0.001; and the results of Example 2 (HFD) on day 10 and day 14 show statistically significant difference with *p*-value less than 0.05, on day 17 and day 19 show statistically significant difference with *p*-value less than 0.01 and on day 21, day 24, day 26 and day 28 show statistically significant difference with *p*-value less than 0.001.

Accordingly, when administering the peptide of Preparation Example 1 for treatment, the dosage of 5 mg/kg does not effectively inhibit the body weight increment of mice with obesity, and the dosages of 15 mg/kg (Example 1) and 50 mg/kg (Example 2) can effectively inhibit the body weight increment of mice with obesity. In other words, by controlling the dosage of the peptide of Preparation Example 1 within a specific range, it demonstrates the therapeutic effect of inhibiting the body weight increment of mice with obesity.

Referring to the results in FIG. 5, which shows the body weight change data of each mouse of the same groups shown in FIG. 4, the body weight change of Comparative Example 1 (high-fat diet) on day 28 is lower than that of Solvent group 2 (high-fat diet), while no statistically significant difference is found between these two groups. In contrast, the body weight changes of Example 1 (high-fat diet) and Example 2 (high-fat diet) on day 28 are much lower than that of Solvent group 2 (high-fat diet), which shows statistically significant difference. Accordingly, by administering a specific range of dosage of the peptide of Preparation Example 1 for treatment, it actually shows the therapeutic effect of inhibiting the body weight increment of mice with obesity.

### Test Example 2: Evaluation of the effect of the combination of the peptide of the present disclosure and liraglutide for treating obesity with animal experiment

### (1) Building mice model with obesity induced by high-fat diet

Male mice with strain of C57BL/6 (purchased from Lingchang Biological Technology Co., Ltd., Shanghai) were adopted, and these mice were at 6 to 7 weeks age. First, theses mice were fed with Rodent Diet #5C02 (purchased from PMI Nutrition International, LLC, IN, USA) for seven days as acclimation phase (acclimation days 0 to 6). Then, high-fat diet (purchased from Research Diets, Inc; product number: D12492; hereinafter referred to as HFD) or normal diet (purchased from Research Diets, Inc; product number: D12450J; hereinafter referred to as ND) were respectively used to feed different groups of mice for 14 weeks (pre-dose days 0 to 98), so the mice fed with high-fat diet were induced to have obesity, and the mice with high-fat diet-induced obesity (DIO) were obtained.

The mice of different groups were housed in polysulfone cages with wood shavings, and animals might be individually housed when a suitable cage-mate was not available. The photoperiod was 12 hours light and 12 hours dark with 7:00 AM to 7:00 PM lights on (might be interrupted for study-related activities); and the temperature was 20°C to 26°C and the relative humidity was 30% to 70%.

During the 14-week induction period (*i.e.*, pre-dose days 0 to 98), mice body weights were measured on pre-dose days 0, 7, 14, 21, 28, 35, 42, 49, 56, 63, 70, 77, 84, 91 and 98, respectively. Besides, on pre-dose day 98, 30 microliters of mice serum were collected and the total cholesterol (TC), triglyceride (TG), low-density lipoprotein cholesterol (LDL-C) and high-density lipoprotein cholesterol (HDL-C) in the serum were measured. Besides, on pre-dose day 99, the mice were scanned by a non-invasive body composition analyzer EchoMRI to obtain the results of whole-body composition, which comprised fat mass and lean mass (without fat weight).

Then, eight mice fed with ND were selected and assigned as a ND group; and based on serum TC, TG, LDL-C and HDL-C levels and body weight measured on pre-dose day 98 and whole-body composition measured on pre-dose day 99, forty mice fed with HFD were selected and randomized into 5 groups as HFD groups (*i.e*., eight mice in each group) for the usage of subsequent experiments, wherein the mice in each group had no obvious differences on the mice body weight, component concentration in the serum and whole-body composition, and low, medium and high body weight values were spread as equally as possible across groups.

### (2) Animal experiment

### (I) Treating conditions

Mice of the ND group and mice having obesity of the HFD group were obtained from the foresaid processes, and these mice were treated with different samples for 28 days (days 0 to 27) for evaluating their therapeutic effects, wherein day 0 was the first day of treatment.

### Samples preparation:

(1) Sample of Solvent 1 (S1): 0.9% sodium chloride for injection (normal saline).
(2) Sample of Solvent 2 (S2): deionized water.
(3) Sample of Liraglutide (L): Liraglutide (purchased from MedChemExpress LLC; product number: HY-P0014) would be formulated to get a liraglutide solution of 2 mg/mL in normal saline (0.9% sodium chloride). Then, 1N of NaOH solution would be slowly added into the liraglutide solution and mixed until receiving a clear solution to obtain a liraglutide stock solution. The liraglutide stock solution would be diluted once daily (before dosing) with normal saline to achieve predetermined concentration of 7.5 µg/ml as the Sample of Liraglutide, which was stored at 2°C to 8°C before use (protected from light).
(4) Sample of Peptide of Preparation Example 1 (P): the peptide was originally stored in a freezer of -60°C to -80°C before use. The peptide was taken out from the freezer and placed at room temperature for about 1 hour before formulation preparation. Then, the required amount of the peptide was weighed out, placed into a suitable container and stirred with a magnetic stirrer after part of required deionized water was added, followed by adding more deionized water to achieve predetermined concentration.

The preparation concentration of above samples for different groups and their preparation frequencies were listed in the following Table 2. The treating conditions of above samples for different groups were listed in the following Table 3. In the following Table 2 and Table 3, "Example 3 (HFD)" and "Example 4 (HFD)" indicated that mice of HFD groups were administered with two active ingredients, including different concentrations of the Sample of Peptide of Preparation Example 1 dissolved in Solvent 2 (oral route) and the Sample of Liraglutide dissolved in Solvent 1 (injection route); "Solvent group 3 (ND)" indicated that mice of ND group were only administered with solvent, including the Sample of Solvent 1 (injection route) and the Sample of Solvent 2 (oral route); "Solvent group 4 (HFD)" indicated that mice of HFD group were only administered with solvent, including the Sample of Solvent 1 and the Sample of Solvent 2; "Control group 2 (HFD)" indicated that mice of HFD group were only administered with the Sample of Liraglutide dissolved in Solvent 1 (injection route); and "Control group 3 (HFD)" indicated that mice of HFD group were only administered with the Sample of Peptide of Preparation Example 1 dissolved in Solvent 2 (oral route). Besides, in the following Table 2 and Table 3, "S1" indicated the Sample of Solvent 1; "S2" indicated the Sample of Solvent 2; "L" indicated the Sample of Liraglutide; "P" indicated the Sample of Peptide of Preparation Example 1; "N" indicated the mouse number; "SC" indicated subcutaneous injection; "PO" indicated administration by oral gavage; "QD" indicated once daily; and "BID" indicated twice daily at 8-hour intervals.

**Table 2: Samples, preparation concentration of samples and preparation frequencies of samples for different groups**

| Group | Sample | Preparation concentration | Preparation frequency |
|---|---|---|---|
| Solvent group 3 (ND) | S1 | -- | once weekly |
| | S2 | -- | once weekly |
| Solvent group 4 (HFD) | S1 | -- | once weekly |
| | S2 | -- | once weekly |
| Control group 2 (HFD) | L (in S1) | 7.5 µg/ml | once daily |
| Control group 3 (HFD) | P (in S2) | 10 mg/ml | once weekly |
| Example 3 (HFD) | P (in S2) | 3 mg/ml | once weekly |
| | L (in S1) | 7.5 µg/ml | once daily |
| Example 4 (HFD) | P (in S2) | 10 mg/ml | once weekly |
| | L (in S1) | 7.5 µg/ml | once daily |

**Table 3: treating conditions for mice of Solvent group 3 (ND), Solvent group 4 (HFD), Example 3 (HFD), Example 4 (HFD), Control group 2 (HFD) and Control group 3 (HFD)**

| Group | Sample | Dosage | N | Administration | |
|---|---|---|---|---|---|
| | | | | Route | Frequency |
| Solvent group 3 (ND) | S1 | 5 mL/kg | 8 | SC | QD |
| | S2 | 5 mL/kg | | PO | BID |
| Solvent group 4 (HFD) | S1 | 5 mL/kg | 8 | SC | QD |
| | S2 | 5 mL/kg | | PO | BID |
| Control group 2 (HFD) | L | 37.5 µg/kg | 8 | SC | QD |
| Control group 3 (HFD) | P | 50 mg/kg | 8 | PO | BID |
| Example 3 (HFD) | P | 15 mg/kg | 8 | PO | BID |
| | L | 37.5 µg/kg | | SC | QD |
| Example 4 (HFD) | P | 50 mg/kg | 8 | PO | BID |
| | L | 37.5 µg/kg | | SC | QD |

### (II) Therapeutic effect evaluation

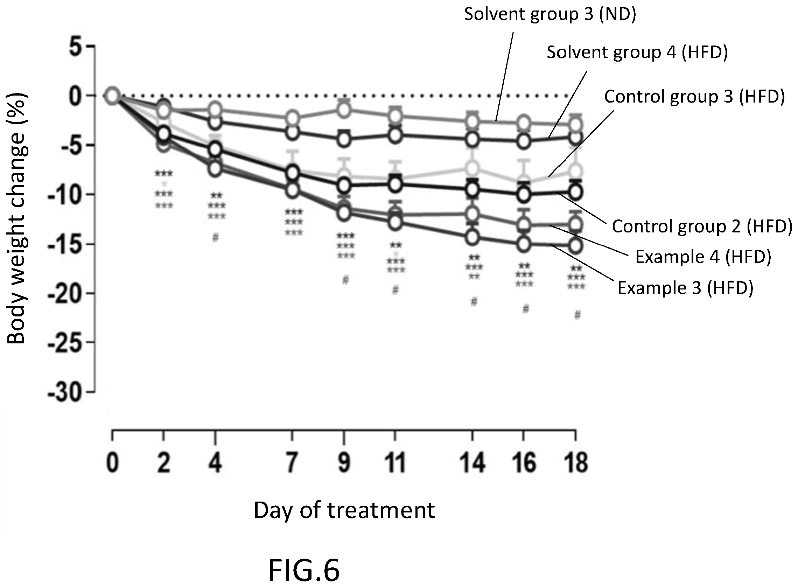

During the process of treating mice of each group according to the treating conditions in Table 3, mice body weights were measured on days 0, 2, 4, 7, 9, 11, 14, 16 and 18, respectively. Then, average body weight on the foresaid each timepoint was calculated, and the body weight change (%) at each timepoint was calculated (the average body weight at each timepoint subtracted the average body weight on day 0, followed by a percentage conversion) for evaluating the effect of obesity treatment, and the results were shown in FIG. 6. The results of each group in FIG. 6 were statistically analyzed by two-way repeated measures ANOVA accompanied with Bonferroni's post hoc test; wherein "*" meant *p*-value less than 0.05, "**" meant *p*-value less than 0.01 and "***" meant *p*-value less than 0.001 when compared to the result of Solvent group 4 (HFD), and "#" meant p-value less than 0.05 when compared to the result of Control group 2 (HFD).

### (III) Discussion for experimental results

Referring to FIG. 6, the mice in Solvent group 3 (ND) and Solvent group (HFD) are treated with S1 and S2, respectively, and their body weight changes are quite similar (with body weight change ranging from 0% to -5%). It indicates that administering mice fed with ND or HFD diet with the solvents (*i.e.*, deionized water and/or 0.9% sodium chloride solution) does not significantly affect the body weight. The foresaid change of body weight should result from normal weight fluctuation or unavoidable measurement errors, etc.

For the mice of Control group 2 (HFD) treated with Liraglutide only, the body weight gradually decreases from day 0 to day 9, and the body weight changes are maintained at about -10% from day 9 to day 18. It indicates that administering mice fed with HFD diet with Liraglutide lowers the body weight and inhibits the body weight increment.

For the mice of Control group 3 (HFD) treated with 50 mg/kg of the peptide of Preparation Example 1 only, the body weight gradually decreases from day 0 to day 9, and the body weight changes are maintained at about -5% to -10% from day 9 to day 18. It indicates that administering mice fed with HFD diet with 50 mg/kg of the peptide of Preparation Example 1 also lowers the body weight and inhibits the body weight increment.

For the mice of Example 3 (HFD) treated with 15 mg/kg of the peptide of Preparation Example 1 and Liraglutide, the body weight continuously decreases from day 0 to day 18, and the body weight change is about -15% on day 18. It indicates that administering mice fed with HFD diet with 15 mg/kg of the peptide of Preparation Example 1 and Liraglutide significantly lowers the body weight and inhibits the body weight increment, even more than Control group 2 (HFD) and Control group 3 (HFD). Specifically, the body weight changes of Example 3 (HFD) from day 2 to day 18 are all significantly lower than those of Solvent group 4 (HFD) with p-value less than 0.001. Besides, body weight changes of Example 3 (HFD) on day 4 and from day 9 to day 18 are significantly lower than those of Control group 2 (HFD) with *p*-value less than 0.05.

For the mice of Example 4 (HFD) treated with 50 mg/kg of the peptide of Preparation Example 1 and Liraglutide, the body weight continuously decreases from day 0 to day 16, and the body weight changes are maintained at about -13% from day 16 to day 18. It indicates that administering mice fed with HFD diet with 50 mg/kg of the peptide of Preparation Example 1 and Liraglutide also significantly lowers the body weight and inhibits the body weight increment. Specifically, the body weight changes of Example 4 (HFD) from day 2 to day 18 are all significantly lower than those of Solvent group 4 (HFD), wherein *p*-values on day 2, day 4, day 7, day 9, day 11, day 16 and day 18 are less than 0.001, and *p*-values on day 14 is less than 0.01.

Besides, according to the results of Control group 2 (HFD), Control group 3 (HFD), Example 3 (HFD) and Example 4 (HFD), in comparison to treating mice fed with HFD diet with the peptide of Preparation Example 1 only, *i.e.*, Control group 2 (HFD), or Liraglutide only, *i.e.*, Control group 3 (HFD), treating with the peptide of Preparation Example 1 and Liraglutide, *i.e.*, Examples 3 and 4 (HFD), demonstrates obviously better effect of lowering the body weight and inhibiting the body weight increment. In other words, by administering the combination of the peptide of Preparation Example 1 and Liraglutide can demonstrate better effects than administering one of them alone.

In summary, within a specific range of dosage, the peptide of the present disclosure actually inhibits the body weight increment caused by high-fat diet, so as to exert therapeutic effect for obesity, thereby providing more therapeutic options for obesity patients.

## Claims

1. A medication for use in treating obesity, **characterized in that** the medication comprises a peptide; wherein the peptide comprises an amino acid sequence set forth in SEQ ID NO: 1, and the effective amount of the peptide is more than 0.5 mg/kg/day.

2. The medication for use according to claim 1, **characterized in that** the effective amount of the peptide is more than or equal to 1 mg/kg/day and less than or equal to 10 mg/kg/day.

3. The medication for use according to claim 2, **characterized in that** the effective amount of the peptide is more than or equal to 1 mg/kg/day and less than or equal to 5 mg/kg/day.

4. The medication for use according to any one of claims 1 to 3, **characterized in that** the medication is administered in combination with a GLP-1 receptor agonist.

5. The medication for use according to claim 4, **characterized in that** an effective amount of the GLP-1 receptor agonist is more than 1 µg/kg/day.

6. The medication for use according to claim 5, **characterized in that** the effective amount of the GLP-1 receptor agonist is more than or equal to 2 µg/kg/day and less than or equal to 20 µg/kg/day.

7. The medication for use according to any one of claims 4 to 6, **characterized in that** the GLP-1 receptor agonist comprises dulaglutide, liraglutide, lixisenatide, semaglutide, tirzepatide, danuglipron, orforglipron, or any combination thereof.

8. The medication for use according to any one of claims 4 to 7, **characterized in that** the GLP-1 receptor agonist comprises liraglutide.

9. The medication for use according to any one of claims 1 to 8, **characterized in that** the obesity is simple obesity.

10. The medication for use according to any one of claims 1 to 9, **characterized in that** the medication further comprises a pharmaceutically acceptable carrier.
